**Europäisches Patentamt**

**European Patent Office**

**Office européen des brevets**

(19)

(11) Numero de publication : **0 125 210**
**B1**

(12)

# FASCICULE DE BREVET EUROPÉEN

(45) Date de publication du fascicule du brevet :
23.12.87

(51) Int. Cl.⁴ : **A 61 M 15/00**

(21) Numéro de dépôt : **84810220.8**

(22) Date de dépôt : **08.05.84**

(54) Appareil pour le traitement des voies respiratoires.

(30) Priorité : **09.05.83 FR 8307846**

(43) Date de publication de la demande :
**14.11.84 Bulletin 84/46**

(45) Mention de la délivrance du brevet :
**23.12.87 Bulletin 87/52**

(84) Etats contractants désignés :
**AT BE CH DE FR GB IT LI LU NL SE**

(56) Documents cités :
FR-A- 1 048 008
FR-A- 2 056 428
GB-A-   526 678
US-A- 2 479 967

(73) Titulaire : **PIERRE FABRE S.A.**
**125, rue de la Faisanderie**
**F-75116 Paris (FR)**

(72) Inventeur : **Kaeser, Charles**
**18, Chemin de Rennier**
**CH-1009 Pully (CH)**

(74) Mandataire : **Nithardt, Roland**
**CABINET ROLAND NITHARDT Rue Edouard Verdan 15**
**CH-1400 Yverdon-les-Bains (CH)**

## Description

La présente invention concerne un appareil pour le traitement des voies respiratoires un courant d'air forcé préchauffé et humidifié, comportant un applicateur prévu pour être placé sur le visage d'un patient, un générateur d'air préchauffé humidifié et un conduit souple pour relier ce générateur à cet applicateur, ledit générateur comprenant un boîtier, un ventilateur, au moins une résistance chauffante, un réservoir pour le liquide à évaporer, et des organes de commande dudit générateur.

On connaît déjà des appareils de ce type, en particulier un appareil inhalateur décrit par le brevet britannique GB-A-526 678 qui comprend deux chambres superposées dont l'une contient un ventilateur et dont l'autre contient des résistances chauffantes. Un plateau disposé dans la partie supérieure de l'appareil, au-dessus des deux chambres superposées, peut recevoir un liquide ou un solide destiné à être évaporé.

Cet appareil ne comporte pas de réservoir d'eau et n'est pas en mesure de produire un courant d'air fortement humidifié ayant une température de sortie déterminée.

On connaît également un autre appareil décrit par la publication française FR-A-2 056 428 qui concerne un appareil inhalateur pourvu d'un petit réservoir définissant un compartiment annulaire de faible capacité dont une des parois est constituée par un élément cylindrique dans l'épaisseur duquel est incrustée une résistance chauffante.

Cet appareil fonctionne comme un évaporateur dépourvu de soufflerie susceptible de créer un courant d'air humidifié. L'air est aspiré par le patient, longe la paroi extérieure du réservoir réalisé en un matériau poreux, et traverse l'ouverture centrale de l'élément cylindrique chauffant.

Cet appareil ne permet pas d'obtenir un courant d'air fortement humidifié, délivré à une température de sortie déterminée.

Afin d'humidifier l'air plus fortement, la publication FR-A-1 048 008 décrit un appareil comportant un organe poreux, constitué par une mèche qui plonge dans un réservoir de liquide et qui est léchée par un courant d'air. Cependant, vu la surface d'évaporation relativement faible, le débit d'air humidifié est très limité. En outre, sa température dépend directement de celle du moteur.

Il existe encore d'autres appareils lourds, encombrants et coûteux, dont l'utilisation est exclusivement réservée aux centres hospitaliers, en raison précisément de leur encombrement et de leur coût. Leur faible diffusion les rend peu efficaces dans la lutte contre les rhinites ou autres affections des voies respiratoires.

La présente invention se propose de pallier ces divers inconvénients en réalisant un appareil efficace, de construction simplifiée, ce qui rend son prix acceptable pour le public et qui satisfait, de par ses caractéristiques, exactement les exigences de base devant être remplies pour assurer une lutte efficace contre les maladies susmentionnées.

Dans ce but, l'appareil selon l'invention est caractérisé en ce que le réservoir pour le liquide à évaporer est étanche et en ce que ledit appareil comporte de plus un organe poreux partiellement plongé dans ce réservoir et léché par le courant d'air et une sonde de température, montée dans le conduit d'air à proximité de l'entrée de l'applicateur et reliée aux organes de commande pour leur transmettre une information de température permettant un réglage de générateur.

A l'intérieur du générateur, une chambre est conçue de préférence de telle manière qu'il existe, entre la paroi intérieure de cette chambre et la surface extérieure de l'organe poreux, un espace étroit qui définit un passage forcé du courant d'air préchauffé balayant la surface extérieure de l'organe poreux. Cet espace ou ce couloir est de préférence très étroit de sorte qu'un maximum d'air est amené en contact étroit avec la surface extérieure de l'organe poreux et se charge d'humidité par ce contact.

Une résistance chauffante est de préférence enroulée autour de l'organe poreux, cette résistance étant disposée au moins partiellement dans la zone du passage forcé du courant d'air préchauffé. Cette résistance assure l'évaporation du film d'eau à la surface extérieure de l'organe poreux.

L'organe poreux se compose de préférence d'un corps cylindrique creux, évasé à sa partie supérieure et fendu à sa base. Le boîtier comporte de préférence un couvercle de remplissage, ménagé en regard de l'extrémité supérieure évasée de l'organe poreux. De cette manière, il est aisé de remplir le réservoir en versant de l'eau à l'intérieur du corps cylindrique creux de l'organe poreux, à sa partie supérieure évasée qui joue le rôle d'un entonnoir. La fente ménagée à sa base permet l'écoulement de l'eau ou de tout autre liquide de traitement à l'intérieur du réservoir.

L'appareil comporte également un bloc d'alimentation électrique et de commande, qui est de préférence rapporté au boîtier contenant le réservoir, l'organe poreux et le générateur d'air préchauffé. Le raccordement électrique peut se faire par des fiches de raccordement ou un connecteur, dont une partie est solidaire du boîtier et l'autre partie du bloc d'alimentation.

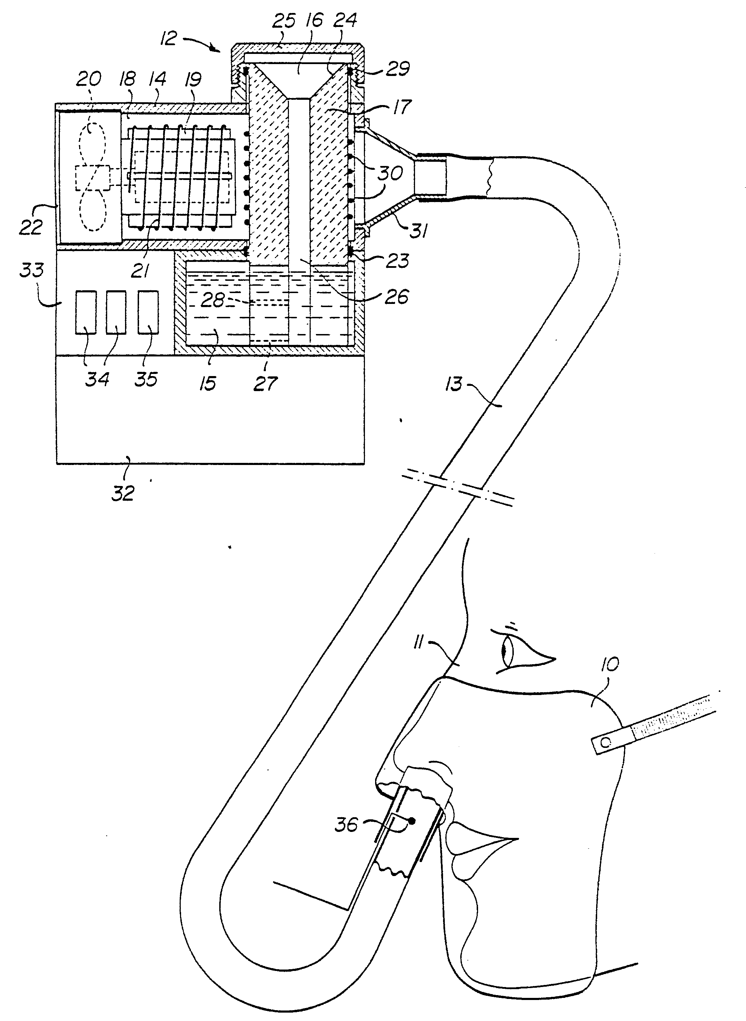

La présente invention sera mieux comprise en référence à la description d'un exemple de réalisation préféré et du dessin annexé qui représente une vue schématique, partiellement coupée de l'appareil selon l'invention.

En référence à la figure, l'appareil pour le traitement des voies respiratoires selon l'invention comporte un applicateur 10 prévu pour être placé sur le visage d'un patient 11, un générateur 12 d'air chaud humidifié et un conduit souple 13 pour relier ce générateur 12 à cet applicateur 10. Le générateur 12 comprend un boîtier 14 compartimenté en trois chambres principales, dont la première 15 constitue un réservoir d'eau, la seconde 16 contient un organe poreux 17, et la troisième 18 contient un moteur électrique 19 entraînant une hélice 20 et une résistance électrique 21, l'ensemble constituant un ventilateur générateur d'air préchauffé. La chambre 18 est obturée à l'arrière par une grille 22 et communique à l'avant avec la chambre 16. La chambre 16 communique, à sa base, avec le réservoir d'eau 15 par l'intermédiaire de l'organe poreux 17 qui traverse la chambre 16 de part en part, et dont l'extrémité inférieure plonge dans le réservoir 15. Un joint 23 assure l'étanchéité entre l'organe poreux 17 et la paroi inférieure de la chambre 16, et la paroi supérieure du réservoir 15.

L'organe poreux 17 a de préférence une forme cylindrique creuse, dont l'extrémité supérieure 24 est évasée, de manière à présenter une forme d'entonnoir. A son extrémité supérieure, la chambre 16 est obturée par un couvercle 25, par exemple vissé sur la paroi latérale de cette chambre, qui permet le remplissage du réservoir. En effet, lorsque le couvercle 25 est retiré, l'utilisateur peut verser de l'eau éventuellement mélangée à un liquide de traitement dans l'entonnoir 24. Ce liquide s'écoule à travers l'alésage central 26 de l'organe poreux 17 dans le réservoir 15. A cet effet, l'organe poreux 17 comporte au moins une fente 27 ou un canal 28 traversant ses parois, ménagé dans sa partie inférieure plongée dans le réservoir 15. Un second joint d'étanchéité 29, disposé au voisinage de l'extrémité supérieure de l'organe poreux 17, permet d'isoler complètement la partie de l'appareil en contact avec l'eau du réservoir du reste de cet appareil. Une résistance électrique 30 entoure la partie centrale de l'organe poreux 17. Un embout conique 31 assure la liaison entre le générateur d'air chaud humidifié et le conduit souple 13.

Les dimensions de la chambre 16 et de l'organe poreux 17 sont telles que l'air préchauffé, refoulé par le ventilateur contenu dans la chambre 18, s'écoule à travers un espace laminaire ménagé entre la surface périphérique de l'organe poreux 17 et la paroi latérale intérieure de la chambre 16. De cette manière, l'air préchauffé refoulé par le ventilateur balaye la surface de l'organe poreux imprégné d'eau et/ou de liquide de traitement et s'humidifie fortement à ce passage.

Un bloc d'alimentation 32, contenant essentiellement un transformateur destiné à alimenter le moteur électrique 19 sous une tension de par exemple vingt-quatre volts, est associé à un bloc de commande 33 pourvu de deux voyants lumineux 34 et d'un interrupteur 35. Une sonde de température 36 est montée dans le conduit 13 à son extrémité voisine de l'applicateur 10 et transmet, au bloc de commande 33, une information de température qui permet un réglage précis de préchauffage et/ou du débit de l'air, pour que l'air chaud et humide, injecté dans les narines du patient, ait une température constante voisine de 43 °C, température à laquelle les microbes responsables de la rhinite, de la bronchite ou de la pharyngite sont détruits.

Cet appareil est particulièrement efficace parce qu'il est compact, de construction simple et, par conséquent, d'un prix de revient abordable, ce qui lui permet d'être diffusé de façon très large.

## Revendications

1. Appareil pour le traitement des voies respiratoires par un courant d'air forcé préchauffé et humidifié, comportant un applicateur (10) prévu pour être placé sur le visage (11) d'un patient, un générateur d'air forcé préchauffé et humidifié (12) et un conduit souple (13) pour relier ce générateur à cet applicateur, ledit générateur comprenant un boîtier (14), un ventilateur (19, 20), au moins une résistance chauffante (21, 30), un réservoir (15) pour le liquide à évaporer, et des organes de commande (33) dudit générateur, caractérisé en ce que le réservoir (15) pour le liquide à évaporer est étanche et en ce que ledit appareil comporte de plus un organe poreux (17) partiellement plongé dans ce réservoir et léché par le courant d'air et une sonde de température (36), montée dans le conduit d'air souple (13) à proximité de l'entrée de l'applicateur (10) et reliée aux organes de commande (33) pour leur transmettre une information de température permettant un réglage du générateur (12).

2. Appareil selon la revendication 1, caractérisé en ce qu'il comprend, à l'intérieur du générateur (12), une chambre (16) conçue de telle manière qu'il existe, entre la paroi intérieure de cette chambre et la surface extérieure de l'organe poreux (17), un espace étroit qui définit un passage forcé du courant d'air préchauffé balayant la surface extérieure de l'organe poreux.

3. Appareil selon la revendication 2, caractérisé en ce qu'il comporte une résistance chauffante (30) enroulée autour de l'organe poreux (17) et disposée au moins partiellement dans la zone de passage forcé du courant d'air préchauffé.

4. Appareil selon la revendication 1, caractérisé en ce que l'organe poreux (17) comprend un corps cylindrique creux évasé à sa partie supérieure (24) et comportant au moins une ouverture (27, 28) à sa base, et en ce que le boîtier comporte un couvercle (25) de remplissage, ménagé en regard de la partie supérieure évasée (24) de l'organe poreux (17).

5. Appareil selon la revendication 1, caractérisé en ce qu'il comporte un bloc d'alimentation électrique (32) et un bloc de commande (33), sur lequel sont fixés au moins un interrupteur (35) et au moins un voyant lumineux (34).

## Claims

1. Apparatus for the treatment of the respiratory passages by a preheated and humidified current of forced air, comprising an applicator (10) intended to be placed on the face (11) of a patient, a generator for preheated and humidified forced air (12) and a flexible duct (13) to connect this generator to this applicator, the said generator comprising a housing (14), a ventilator (19, 20), at least one heating resistance (21, 30), a tank (15) for the liquid to be evaporated, and controls (33) for the said generator, characterised in that the tank (15) for the liquid to be evaporated is watertight and in that the said apparatus has more than one porous component (17) partially inserted into the tank and licked by the air current and a temperature probe (36), mounted in the flexible air duct (13) in the vicinity of the entrance to be applicator (10) and connected to the controls (33) so as to transmit to them information regarding temperature which enables the generator (12) to be regulated.

2. Apparatus according to Claim 1, characterised in that, inside the generator (12), it has a chamber (16) designed so that there is, between the internal wall of this chamber and the outer surface of the porous component (17), a narrow space which defines a forced passage of the preheated air current sweeping the outer surface of the porous component.

3. Apparatus according to Claim 2, characterised in that it has a heating resistance (30) coiled around the porous component (17) and disposed at least partially in the zone of the forced passage of the preheated air current.

4. Apparatus according to Claim 1, characterised in that the porous component (17) comprises a hollow cylindrical body which is funnel-shaped at its upper part (24) and comprising at least one opening (27, 28) at its base, and in that the housing has a filling cover (25) arranged facing the upper funnel-shaped part (24) of the porous component (17).

5. Apparatus according to Claim 1, characterised in that it comprises an electrical supply unit (32) and a control unit (33), on which are fixed at least one switch (35) and at least one signal lamp (34).

**Patentansprüche**

1. Gerät für die Behandlung der Atemwege durch eine Luftströmung, die mit einem Druck gefördert wird und die vorgewärmt und befeuchtet ist, bestehend aus einer Maske (10), die vorgesehen ist, auf das Gesicht (11) eines Patienten aufgelegt zu werden, aus einem Erzeuger von Luft (12), die vorgewärmt und befeuchtet mit Druck gefördert wird und aus einem biegsamen Rohr (13), das den Erzeuger mit der Maske verbindet, wobei der Erzeuger aus einem Gehäuse (14), einem Ventilator (19, 20), mindestens einem Heizwiderstand (21, 30), einem Behälter (15) für die zu verdampfende Flüssigkeit und aus Organen für die Steuerung (33) des Erzeugers besteht, dadurch gekennzeichnet, daß der Behälter (15) für die Flüssigkeit, die zu verdampfen ist, dicht ist und daß das Gerät ferner ein poröses Teil (17) enthält, der teilweise in den Behälter eintaucht und von der Luftströmung umströmt wird und einen Temperaturfühler (36), der in dem biegsamen Rohr für Luft (13) in der Nähe des Einlaufs in der Maske (10) eingebaut und mit den Organen für die Steuerung (33) zur Weiterleitung einer Angabe über die Temperatur für eine Regelung des Erzeugers (12) verbunden ist.

2. Gerät nach Anspruch 1, dadurch gekennzeichnet, daß es in dem Inneren des Erzeugers (12) eine Kammer (16) gibt, die derart gestaltet ist, daß zwischen der inneren Wandung der Kammer (16) und der äußeren Oberfläche des porösen Teils (17) ein enger Raum als zwangsweiser Durchgang für die Strömung von vorgewärmter Luft vorhanden ist, die die äußere Oberfläche des porösen Teils umströmt.

3. Gerät nach Anspruch 2, dadurch gekennzeichnet, daß es einen Heizwiderstand (30) enthält, der um den porösen Teil (17) gewickelt und mindestens teilweise in dem Bereich des zwangsweisen Durchgangs der Strömung der vorgewärmten Luft angeordnet ist.

4. Gerät nach Anspruch 1, dadurch gekennzeichnet, daß der poröse Teil (17) aus einem zylindrischen hohlen Körper besteht, der an seinem oberen Teil (24) ausgeweitet ist, und an seiner Basis eine Öffnung (27, 28) enthält und daß das Gehäuse einen Deckel (25) für das Füllen enthält, der gegenüber dem oberen ausgeweiteten Teil (24) des porösen Teils angebracht ist.

5. Gerät gemäß dem Anspruch 1, dadurch gekennzeichnet, daß es eine Einheit für die elektrische Versorgung (32) und eine Einheit für die Steuerung (33) enthält, auf der mindestens ein Schalter (35) und mindestens eine Leuchte (34) für eine Anzeige befestigt sind.